# EUROPÄISCHE PATENTSCHRIFT

(11) **EP 1 930 923 B1**
(45) Veröffentlichungstag und Bekanntmachung des Hinweises auf die Patenterteilung: **28.03.2012**
(21) Anmeldenummer: 07120046.3
(22) Anmeldetag: 06.11.2007
(51) Int. Cl.: H01H 3/14, G06F 3/033, G05G 1/30

(54) **Schalteinrichtung für medizinische oder chirurgische Geräte**
Switching device for medical or surgical instruments
Dispositif de commutation pour appareils médicaux ou chirurgicaux

(30) Priorität: 07.12.2006 DE 102006057682
(43) Veröffentlichungstag der Anmeldung: 11.06.2008
(73) Patentinhaber: Aesculap AG, 78532 Tuttlingen (DE)
(72) Erfinder: Gassner, Stefan, 78194, Immendingen-Hattingen (DE)
(74) Vertreter: Hoeger, Stellrecht & Partner Patentanwälte

(56) Entgegenhaltungen:
- DE-A1- 19 726 234
- US-A- 5 441 042

## Beschreibung

Die Erfindung betrifft eine Schalteinrichtung für medizinische oder chirurgische Geräte.

Zur Steuerung von derartigen Geräten oder Software, insbesondere im Operationssaal bei chirurgischen Operationen, werden häufig elektromechanische Fußschalter mit mehreren Schaltelementen verwendet, die beispielsweise Pedale, Handtaster oder Verstellknöpfe aufweisen. So ist es bekannt, unterhalb des Operationstisches Fußschalter zu positionieren, die über Kabel oder über eine drahtlose Übertragungslinie mit einer Datenverarbeitungseinrichtung verbunden sind. Diese Fußschalter sind häufig verschiebbar, so dass die Bedienungsperson, beispielsweise ein operierender Arzt, diese Fußschalter an die jeweilige Einsatzposition ziehen kann, von der ausgehend er gerade operiert. Andererseits stören diese Fußschalter auch den Arbeitsablauf, so dass häufig die Fußschalter von der Bedienungsperson weggeschoben werden, wenn sie nicht benötigt werden. Es ist dann notwendig, diese Fußschalter zu suchen, wenn sie wieder eingesetzt werden müssen, und dies bedeutet eine Unterbrechung des Arbeitsablaufes.

Bei derartigen Operationen bewegt sich die Bedienungsperson im Raum um den Operationstisch herum, und es ist daher schwierig, Schalteinrichtungen so anzuordnen, dass sie von den unterschiedlichen Arbeitspositionen der Bedienungsperson aus schnell und unkompliziert bedient werden können.

DE 197 26 234 A1 offenbart eine Schalteinrichtung gemäß dem Oberbegriff des Anspruchs 1.

Es ist daher Aufgabe, eine Schalteinrichtung für medizinische oder chirurgische Geräte zu schaffen, die es einer Bedienungsperson ermöglicht, auch bei gegebenenfalls häufigem Ortswechsel in einfachster Weise Schaltsignale zu erzeugen.

Diese Aufgabe wird erfindungsgemäß gelöst durch eine Schalteinrichtung für medizinische oder chirurgische Geräte, die gekennzeichnet ist durch eine flächige Fußmatte mit über ihre Fläche verteilten Sensoren, die ein der Position eines Fußes einer Bedienungsperson auf der Fußmatte entsprechendes elektrisches Signal erzeugen, wenn sich der Fuß bei dem jeweiligen Sensor befindet, und durch eine mit der Fußmatte verbundene Datenverarbeitungseinrichtung, die so programmiert ist, dass sie bei Aktivierung durch ein Aktivierungssignal die momentane Position des Fußes auf der Fußmatte als Ausgangsposition speichert, mindestens einen Schaltbereich der Fußmatte, der relativ zu der Ausgangsposition eine vorgegebene Lage aufweist, auswählt und ein Schaltsignal erzeugt, wenn der Fuß sich bei einem Sensor in dem Schaltbereich befindet.

Eine derartige flächige Fußmatte kann im Arbeitsbereich der Bedienungsperson ausgelegt werden, so dass sich die Bedienungsperson auf dieser Fußmatte bewegt. Die jeweilige momentane Position eines Fußes der Bedienungsperson wird durch entsprechend über die Fußmatte verteilte Sensoren festgestellt und an die Datenverarbeitungseinrichtung gemeldet. Diese Signale werden nur von der Datenverarbeitungseinrichtung verwendet, wenn dieser ein Aktivierungssignal zugeführt wird. Ist dies der Fall, so wird die momentane Position des Fußes festgestellt und dient zur Definierung einer Ausgangsposition, diese Ausgangsposition wird in der Datenverarbeitungseinrichtung gespeichert. Ausgehend von dieser gespeicherten Ausgangsposition werden auf der Fußmatte ein oder vorzugsweise mehrere Schaltbereiche definiert, die relativ zu dieser Ausgangsposition auf der Fußmatte eine bestimmte Lage und eine bestimmte Größe aufweisen. Wenn nach einer solche Aktivierung durch ein Aktivierungssignal in einem solchen Schaltbereich die Anwesenheit eines Fußes des Operateurs festgestellt wird, wird das von dem die Anwesenheit des Fußes feststellenden Sensor gelieferte elektrische Signal von der Datenverarbeitungseinrichtung als Schaltsignal interpretiert und genutzt. Einem Schaltbereich ist dabei eine bestimmte Schaltfunktion zugeordnet, es kann beispielsweise ein Schaltbereich für das Einschalten eines Gerätes und ein anderer Schaltbereich für das Ausschalten dieses Gerätes vorgesehen sein. Die Bedienungsperson kann also nach der Aktivierung einfach dadurch ein Gerät ein- bzw. ausschalten, dass der Fuß ausgehend von der Ausgangsposition bei Erzeugung des Aktivierungssignals in den Schaltbereich gesetzt wird, der für das Einschalten bzw. das Ausschalten zuständig ist. Die Bewegung ist dabei immer gleich, da diese Schaltbereiche bei jedem Aktivierungssignal neu definiert werden, und zwar derart, dass sie von der Momentanposition des Fußes beim Aktivierungssignal ausgehend immer dieselbe Lage und dieselbe Größe haben. Die Bedienungsperson weiß also, dass nach einem Aktivierungssignal beispielsweise das Einschaltsignal immer 15 Zentimeter vor der Momentanposition des Fußes liegt und das Ausschaltsignal entsprechend 15 Zentimeter hinter dieser Momentanposition, wenn die Einschalt- und Ausschaltbereiche entsprechend definiert sind. Dies trifft an jeder Stelle der Fußmatte zu, ist also unabhängig von der tatsächlichen Position der Bedienungsperson auf der Fußmatte.

Bei einer einfachen Anordnung dieser Art, bei der beispielsweise nur ein Einschalt- und ein Ausschaltbereich vorgesehen sind, benötigt die Bedienungsperson nicht unbedingt eine Visualisierung der geometrischen Verhältnisse. Es ist jedoch bei einer bevorzugten Ausführungsform der Erfindung vorgesehen, dass die Schalteinrichtung eine Anzeigeeinrichtung aufweist, die den Schaltbereich oder gegebenenfalls die Schaltbereiche und die jeweilige momentane Position des Fußes der Bedienungsperson anzeigt. Die jeweilige momentane Position des Fußes kann dabei in der Art eines Cursors auf der Anzeige entsprechend der Bewegung des Fußes auf der Fußmatte dargestellt werden, während die Schaltbereiche stationär dargestellt werden. Der Operateur kann daher an der Anzeige verfolgen, wie sich der Fuß ausgehend von der Ausgangsposition auf der Fußmatte in Richtung auf einen Schaltbereich bewegt und diesen erreicht. Dies ist insbesondere dann von Vorteil, wenn eine größere Anzahl von Schaltbereichen vorgesehen sind, die um die Ausgangsposition herum in bestimmter Verteilung angeordnet sind und die von der Bedienungsperson zur Erzeugung eines Schaltsignals selektiv mit dem Fuß angefahren werden müssen.

Die Anzeigeeinrichtung kann so ausgebildet sein, dass sie immer im gesamten Anzeigefeld die Umgebung der Ausgangsposition darstellt, es kann aber auch gemäß einer bevorzugten Ausführungsform vorgesehen sein, dass der Schaltbereich oder gegebenenfalls die Schaltbereiche in einem Ausschnitt der Anzeige dargestellt werden und dass die Lage des Ausschnittes auf der Anzeige der durch die Ausgangsposition definierten Lage der Schaltbereiche auf der Fußmatte entspricht. Wenn sich die Bedienungsperson also am linken Ende der Fußmatte befindet, wird der Ausschnitt auch am linken Rand der Anzeige dargestellt. Damit wird der Bedienungsperson nicht nur die relative Lage des Fußes zu den Schaltbereichen angezeigt, sondern auch seine Position auf der Fußmatte.

Nach der Erzeugung des Aktivierungssignales bleibt die Ausgangsposition fest. Es kann jedoch gemäß einer bevorzugten Ausführungsform ein Verschiebeschaltbereich vorgesehen sein, der ein Verschiebesignal erzeugt, wenn sich der Fuß der Bedienungsperson von diesem Verschiebeschaltbereich ausgehend auf der Fußmatte verschiebt, und die Datenverarbeitungseinrichtung kann die Ausgangsposition und damit die Schaltbereiche entsprechend der Bewegung des Fußes von dem Verschiebeschaltbereich an eine andere Stelle der Fußmatte verschieben und mit den neuen Positionsdaten speichern. Die Bedienungsperson hat also die Möglichkeit, durch Bewegung des Fußes von dem Verschiebeschaltbereich aus in einer beliebigen Richtung und über eine beliebige Strecke die Ausgangsposition auf der Fußmatte entsprechend zu verschieben. Damit können die Schaltbereiche einem Positionswechsel der Bedienungsperson auf der Fußmatte folgen.

Es ist günstig, wenn ein Schaltbereich vorgesehen ist, der ein Deaktivierungssignal erzeugt, durch das die Erzeugung von Schaltsignalen beendet wird. Die Bedienungsperson kann damit die Schaltfunktion unterbrechen, so dass eine Bewegung der Bedienungsperson auf der Fußmatte keine Schaltsignale erzeugt. Falls die Bedienungsperson erneut eine Schaltfunktion ausführen will, muss dazu ein erneutes Aktivierungssignal erzeugt werden.

Es kann auch vorgesehen sein, dass die Datenverarbeitungseinrichtung nach Ablauf einer vorgegebenen Zeitspanne ausgehend von dem Aktivierungssignal ein Deaktivierungssignal erzeugt, durch das die Erzeugung von Schaltsignalen beendet wird. Die Bedienungsperson weiß dann, dass innerhalb dieser Zeitspanne nach dem Aktivierungssignal eine Bewegung des Fußes in einen Schaltbereich hinein eine Schaltfunktion auslöst, nach Ablauf dieser Zeitspanne ist die Bedienungsperson aber in der Bewegung des Fußes völlig frei, ohne dass Schaltsignale erzeugt werden.

Das Aktivierungssignal kann beispielsweise von einem akustischen Sensor erzeugt werden, insbesondere durch ein Spracherkennungssystem.

Bei einer anderen bevorzugten Ausführungsform ist vorgesehen, dass das Aktivierungssignal von der Datenverarbeitungseinrichtung erzeugt wird, wenn ihr von einem einzigen Sensor an einer beliebigen Stelle der Fußmatte nacheinander Signale in einer bestimmten Folge zugeführt werden, beispielsweise in einer Folge aus zwei Signalen innerhalb eines kurzen Zeitraums ("Doppelklick").

Solange sich die Bedienungsperson bei ihrer Arbeit auf der Fußmatte beliebig bewegt, wird kein Aktivierungssignal erzeugt, wenn die Bedienungsperson aber an einer beliebigen Stelle eine vorgegebene Folge von Fußpositionen einnimmt, wird dies als Aktivierungssignal erkannt und führt dann dazu, dass bei der weiteren Bewegung des Fußes in einen Schaltbereich hinein ein Schaltsignal erzeugt wird.

Es kann vorgesehen sein, dass die Datenverarbeitungseinrichtung nach dem Aktivierungssignal nur Signale von Sensoren berücksichtigt, bei denen sich nach dem Aktivierungssignal die Anwesenheit des Fußes der Bedienungsperson geändert hat. Dadurch wird sichergestellt, dass Schaltsignale nur mit einem bewusst bewegten Fuß der Bedienungsperson erzeugt werden, nicht jedoch durch einen zweiten Fuß, der im Wesentlichen nach der Aktivierung unbewegt auf der Fußmatte bleibt.

Die Sensoren in der Fußmatte können nach unterschiedlichen physikalischen Prinzipien arbeiten, wesentlich ist lediglich, dass die Anwesenheit des Fußes an einer bestimmten Stelle der Fußmatte festgestellt wird. Beispiele für derartige Sensoren sind Drucksensoren, Ultraschallsensoren, kapazitive Sensoren, Hall-Sensoren oder analog-resistive Sensoren.

Insbesondere kann auch vorgesehen sein, dass die Sensoren auf die Anwesenheit von Schaltelementen ansprechen und dass ein solches Schaltelement an einem Fuß der Bedienungsperson angeordnet ist. Es kann sich beispielsweise bei einem solchen Schaltelement um einen Permanentmagneten handeln, der magnetfeldempfindliche Sensoren in der Fußmatte aktiviert. Auf diese Weise ist sichergestellt, dass nur ein Fuß der Bedienungsperson derartige Signale bei den Sensoren erzeugt, nicht dagegen der andere.

Die Größe der Schaltbereiche auf der Fußmatte und/oder deren relative Lage zur Ausgangsposition können einstellbar sein, so dass die Bedienungsperson diese Parameter frei auswählen kann.

Insbesondere kann ein Einstellschaltbereich auf der Fußmatte vorgesehen sein, der ein Verschiebesignal erzeugt, wenn sich der Fuß der Bedienungsperson von diesem Einstellschaltbereich ausgehend auf der Fußmatte verschiebt. In diesem Falle verstellt die Datenverarbeitungseinrichtung die Größe der Schaltbereiche und/oder deren relative Lage zur Ausgangsposition entsprechend der Bewegung des Fußes von dem Einstellschaltbereich an eine andere Stelle der Fußmatte.

Ein solcher Einstellschaltbereich kann auch dazu verwendet werden, dass die Datenverarbeitungseinrichtung die Größe eines Parameters eines chirurgischen Gerätes entsprechend der Bewegung des Fußes von dem Einstellschaltbereich an eine andere Stelle der Fußmatte verstellt, beispielsweise die Drehzahl eines Motors, die Stärke eines Saugaggregates, die Ausgangsspannung eines Hochfrequenzgenerators etc.

Gemäß einem bevorzugten Ausführungsbeispiel ist vorgesehen, dass die Datenverarbeitungseinrichtung derart programmiert ist, dass sie den oder die Schaltbereiche entsprechend der Verschiebung des Fußes in eine neue momentane Position auf der Fußmatte verschiebt, wenn der Fuß über einen bestimmten Zeitraum an der neuen momentanen Position verweilt. Dadurch wird es dem Operateur möglich, seine Position zu wechseln und auch in der neuen Position die Schaltbereiche an der gewohnten Position zu finden, wenn er die neue Position über einen bestimmten Zeitraum einnimmt. Bei einem schnellen Wechsel dagegen bleiben die Schaltbereiche an der alten Position, so dass nicht bei dem Wechsel unbeabsichtigt Schalt- oder Einstellsignale erzeugt werden.

Gemäß einer weiteren bevorzugten Ausführungsform umfasst die Datenverarbeitungseinrichtung eine Auswahleinrichtung zur selektiven Ansteuerung und Versorgung mit Schalt- und/oder Einstellsignalen verschiedener chirurgischer Geräte. Durch Betätigen der Auswahleinrichtung kann man also auswählen, welches Gerät durch die Datenverarbeitungseinrichtung mit den Schalt- und/oder Einstellsignalen versorgt wird, die der Operateur an der Fußmatte erzeugt.

Insbesondere ist es dabei vorteilhaft, wenn die Datenverarbeitungseinrichtung für verschiedene chirurgische Geräte verschiedene Anordnungen, Größen und/oder Anzahl von Schaltbereichen auf der Fußmatte definiert, so dass der Operateur für ein bestimmtes Gerät eine nur für dieses Gerät zutreffende Anordnung von Schaltbereichen auf der Fußmatte findet.

Insbesondere kann dabei vorgesehen sein, dass der Auswahleinrichtung selbst ein oder mehrere eigene Schaltbereiche der Fußmatte zugeordnet sind, so dass der Operateur durch Betätigung dieses Schaltbereichs oder dieser Schaltbereiche die Auswahleinrichtung betätigen und damit von einem chirurgischen Gerät auf das andere umschalten kann. Er kann also selbst in diesem Falle auch die spezielle Anordnung der Schaltbereiche für ein bestimmtes Gerät beeinflussen.

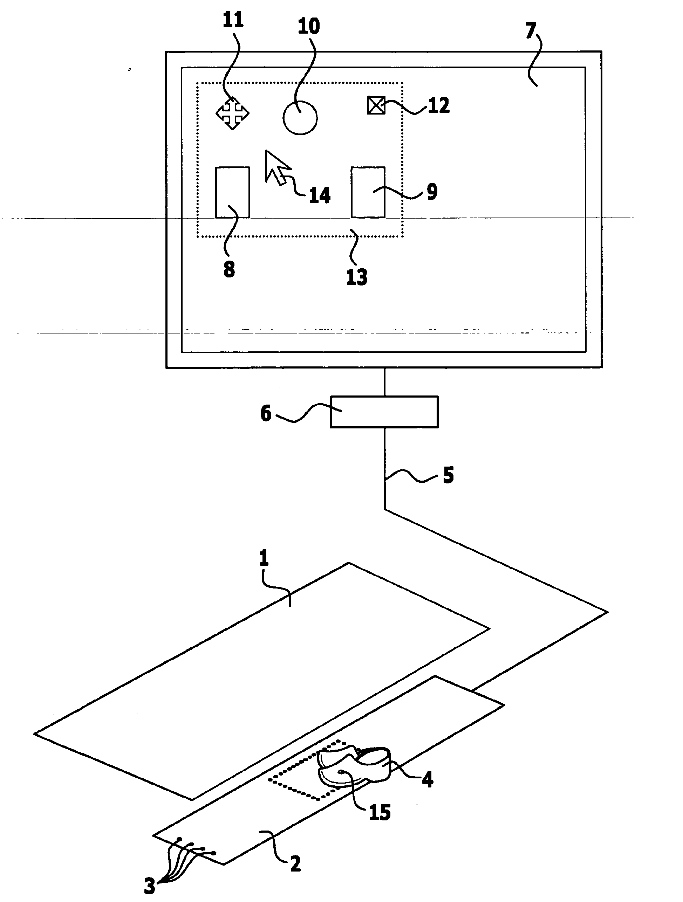

Die nachfolgende Beschreibung bevorzugter Ausführungsformen der Erfindung dient im Zusammenhang mit der Zeichnung der näheren Erläuterung. Die Zeichnung zeigt eine schematische Ansicht der Schalteinrichtung in einem Operationssaal mit einer Sensor-Fußmatte und einer Anzeige.

In einem Operationssaal steht ein Operationstisch 1, auf dem ein Patient operiert wird. Die Operation wird von einem Arzt durchgeführt, der sich bei der Operation längs der Kanten des Operationstisches 1 frei bewegt, um von allen Seiten her Zugang zum Patienten zu haben.

In dem Bereich, in dem der Operateur sich bewegt, liegt auf dem Boden des Operationssaals eine flächige, dünne Fußmatte 2, auf der der Operateur bei seiner Tätigkeit steht und sich gegebenenfalls bewegt.

Diese Fußmatte ist mit einer großen Anzahl von Sensoren 3 versehen, die gleichmäßig über die gesamte Fläche der Fußmatte 2 verteilt sind. In der Zeichnung sind nur wenige dieser Sensoren 3 dargestellt.

Diese Sensoren 3 sind so ausgebildet, dass sie die Position eines Fußes des Operateurs an einer bestimmten Stelle der Fußmatte 2 feststellen können. In der Zeichnung sind stellvertretend für die Füße des Operateurs Schuhe 4 dargestellt, deren Position durch die Sensoren 3 festgestellt wird.

Die Sensoren können dabei unterschiedlich ausgebildet sein und nach verschiedenen physikalischen Prinzipen arbeiten, beispielsweise kann es sich dabei handeln um Drucksensoren, um Ultraschallsensoren, um kapazitive oder analog-resistive Sensoren, um Hall-Sensoren etc. Die Sensoren können unmittelbar auf den Fuß oder den Schuh des Operateurs ansprechen oder aber auch auf ein Schaltelement, das an einem Fuß des Operateurs befestigt ist, beispielsweise einen Permanentmagneten 15.

Die Dichte der Sensoren 3 auf der Fußmatte 2 ist so gewählt, dass die Position des Fußes oder des Schuhs 4 mit der gewünschten Genauigkeit festzustellen ist, beispielsweise können die Sensoren 3 untereinander Abstände zwischen 0,5 cm und 5,0 cm aufweisen.

Vorzugsweise ist es natürlich vorzusehen, dass die Dichte der Sensoren 3 relativ hoch ist. Insbesondere in diesem Falle ist es dann allerdings nicht unbedingt notwendig, dass für alle Funktionen immer alle Sensoren aktiv sind. So ist es beispielsweise möglich, für reine Schaltvorgänge, also beispielsweise Einschalten und Ausschalten, nur jeden zweiten oder jeden dritten Sensor 3 im Raster zu aktivieren, dagegen ist es günstig, die maximale Empfindlichkeit auszunützen und alle Sensoren 3 zu aktivieren, wenn ein Signal erzeugt werden soll, das der Veränderung der Position des Fußes auf der Fußmatte entspricht, also beispielsweise ein Signal zum Verstellen der Größe der Schaltbereiche oder einer Arbeitsgröße eines chirurgischen Gerätes.

Alle Sensoren 3 sind über eine Leitung 5 oder auch über eine drahtlose Übertragungslinie mit einer Datenverarbeitungseinrichtung 6 verbunden, der auch eine Anzeigeeinrichtung 7 zugeordnet ist, beispielsweise in Form eines Bildschirmes. Die Anzeigeeinrichtung 7 könnte auch eine vom Operateur getragene Brille sein, in die von der Datenverarbeitungseinrichtung Bilddaten eingespielt werden.

Die Datenverarbeitungseinrichtung 6 empfängt Signale von den Sensoren 3, in deren Empfindlichkeitsbereich sich ein Fuß oder Schuh 4 befinden. Normalerweise haben diese Signale keine Wirkung in der Datenverarbeitungseinrichtung 6, so dass sich der Operateur frei auf der Fußmatte 2 bewegen kann, ohne dass die dabei von den Sensoren 3 erzeugten und an die Datenverarbeitungseinrichtung 6 übertragenen Signale Auswirkungen haben.

Es besteht jedoch die Möglichkeit, die Datenverarbeitungseinrichtung 6 durch ein Aktivierungssignal zu aktivieren. Dieses Aktivierungssignal kann auf unterschiedliche Weise von dem Operateur oder einer Hilfsperson erzeugt werden, beispielsweise durch ein akustisches Signal, insbesondere ein Sprachsignal des Operateurs. Es ist auch möglich, dass ein bestimmte Signalfolge an einem Sensor 3 als Aktivierungssignal von der Datenverarbeitungseinrichtung 6 erkannt wird. Beispielsweise kann der Operateur einen Fuß in einer bestimmte Abfolge kurz hintereinander an eine bestimmte Stelle der Fußmatte bewegen, so dass der an dieser bestimmten Stelle angeordnete Sensor 3 eine entsprechende Folge von Signalen erzeugt. Dabei kann es sich insbesondere um einen sogenannten Doppelklick handeln, also um zwei kurz nacheinander auftretende Signale innerhalb eines kurzen Zeitraumes. Dies kann von jeder Stelle der Fußmatte 2 aus erfolgen, da an jeder Stelle der Fußmatte 2 Sensoren 3 angeordnet sind, die derartige Anwesenheitssignale des Fußes erzeugen.

Wenn die Datenverarbeitungseinrichtung 6 in dieser Weise durch ein Aktivierungssignal aktiviert wird, stellt sie fest, an welcher Stelle der Fußmatte 2, also bei welchem Sensor 3 der Fußmatte 2 sich der Fuß im Augenblick des Aktivierungssignals befindet. Diese Position des Fußes oder Schuhs 4 wird von der Datenverarbeitungseinrichtung 6 als Ausgangsposition gespeichert und die Datenverarbeitungseinrichtung 6 definiert daraufhin auf der Fußmatte 2 einen oder mehrere Schaltbereiche 8, 9, 10, 11, 12, die alle eine bestimmte flächige Ausdehnung haben und deren Lage relativ zu der Ausgangsposition vorgegeben ist. In dem dargestellten Ausführungsbeispiel werden fünf derartige Schaltbereiche 8 bis 12 definiert, die in vorgegebener Weise um die Ausgangsposition herum gruppiert sind.

In jedem Schaltbereich sind Sensoren 3 angeordnet, und zwar mindestens einer, vorzugsweise jedoch eine größere Anzahl von Sensoren 3. Wenn die Sensoren eines Schaltbereiches durch die Anwesenheit des Fußes in diesem Schaltbereich erregt werden, senden sie ein entsprechendes Signal an die Datenverarbeitungseinrichtung, diese erkennt diese Signale als Signale, die einem bestimmten Schaltbereich zugeordnet sind und somit eine bestimmte Schaltfunktion auslösen. Es kann sich beispielsweise um ein Einschaltsignal oder ein Ausschaltsignal für ein an die Datenverarbeitungseinrichtung angeschlossenes Gerät handeln.

Der Operateur kann damit an jeder Stelle der Fußmatte 2 eine Ausgangsposition festlegen und weiß, dass ausgehend von dieser Ausgangsposition der Schaltbereich für eine bestimmte Funktion immer in derselben Richtung und im selben Abstand angeordnet ist und dieselbe Größe aufweist. Es ist für ihn daher ohne weiteres möglich, ausgehend von der Ausgangsposition den gewünschten Schaltbereich zu treffen und allein durch die Anwesenheit des Fußes ein Schaltsignal zu erzeugen.

Dies kann im einfachsten Falle auch ohne Anzeige erfolgen, es ist aber natürlich vorteilhaft, wenn die Lage der Schaltbereiche 8 bis 12 auf der Anzeigeeinrichtung 7 dargestellt wird. Außerdem ist es dabei vorteilhaft, wenn die Momentanlage des Fußes auf der Fußmatte 2, die durch Signale der Sensoren 3 jederzeit von der Datenverarbeitungseinrichtung 6 feststellbar ist, zusätzlich auf der Anzeigeeinrichtung 7 z.B. in Form eines Cursors 14 dargestellt ist. Der Operateur kann daher an der Anzeige unmittelbar sehen, wie der Fuß relativ zu den Schaltbereichen steht und kann die Bewegung des Fußes in Richtung auf einen bestimmten Schaltbereich verfolgen.

Die Anzeige könnte so gewählt sein, dass auf der gesamten Fläche der Anzeigeeinrichtung 7 immer in gleicher Weise die Umgebung der Ausgangsposition dargestellt wird, dann befinden sich die Schaltbereiche immer in derselben Position und es bewegt sich lediglich der die Fußposition anzeigende Cursor 14.

Bei der Darstellung in der Zeichnung ist eine andere Möglichkeit gewählt. Es wird nämlich die Umgebung der Ausgangsposition in einem Ausschnitt 13 dargestellt, und dieser Ausschnitt 13, der kleiner ist als die gesamte Anzeigefläche der Anzeigeeinrichtung 7, wird auf der Anzeigeeinrichtung 7 entsprechend der Lage der Ausgangsposition auf der Fußmatte 2 platziert. Wenn also die Ausgangsposition sich nahe des linken Randes der Fußmatte befindet, wird auch der Ausschnitt 13 entsprechend am linken Rand der Anzeigeeinrichtung 7 positioniert etc., so dass der Operateur an der Lage des Ausschnittes 13 seine eigene Position auf der Fußmatte 2 zusätzlich erkennen kann.

In dem dargestellten Ausführungsbeispiel sind Schaltbereiche 8, 9, 10 für bestimmte Schaltfunktionen dargestellt, beispielsweise für das Einschalten, das Ausschalten eines Gerätes oder die Umschaltung von einer Betriebsart in die andere.

Zusätzlich ist ein Schaltbereich 11 vorgesehen, der als Verschiebeschaltbereich ausgebildet ist. Wenn nach einem Aktivierungssignal der Fuß des Operateurs in diesem Schaltbereich 11 festgestellt wird und wenn er sich anschließend von der ursprünglichen Position ausgehend an eine andere Stelle der Fußmatte 2 bewegt, dann wird dieser Verschiebeweg des Fußes von der Datenverarbeitungseinrichtung 6 festgestellt und verwendet, um entsprechend der Größe und/oder Richtung der Verschiebung eine Verstellung von bestimmten Größen vorzunehmen. Bei diesen Größen kann es sich beispielsweise um die Positionsdaten der Ausgangsposition handeln, die nach dem Aktivierungssignal gespeichert worden ist. Der Operateur hat damit die Möglichkeit, diese Ausgangsposition zu verändern, die Ausgangsposition wird dabei synchron und in gleicher Weise wie die Bewegung des Fußes auf der Fußmatte verschoben und in der vom Operateur gewählten neuen Position wieder gespeichert. Damit hat der Operateur die Möglichkeit, die Lage der Schaltbereiche an seine momentane Position anzupassen, wenn der Verschiebeweg relativ klein ist.

Mit einem derartigen Verschiebesignal können auch andere Größen verstellt werden, beispielsweise die Größe der Schaltbereiche oder die Abstände der Schaltbereiche zu der Ausgangsposition. Damit kann sich der Operateur individuell diese Größen jederzeit einstellen, indem er lediglich nach Aktivierung einen entsprechenden Schaltbereich mit dem Fuß anfährt und den Fuß aus diesem Schaltbereich bis zu einer neuen Position auf der Fußmatte bewegt.

Der Schaltbereich 12 ist als Deaktivierungsbereich ausgebildet, d.h. eine Positionierung des Fußes im Schaltbereich 12 führt dazu, dass die Datenverarbeitungseinrichtung 6 die von den Sensoren 3 gelieferten Signale nicht mehr als Schaltsignale bearbeitet. Der Operateur kann sich damit nach Betätigung des Deaktivierungs-Schaltbereiches 12 wieder frei auf der Fußmatte bewegen, ohne dass Schaltsignale erzeugt werden.

Es könnte auch vorgesehen sein, dass die Datenverarbeitungseinrichtung 6 selbsttätig diese Deaktivierung vornimmt, und zwar beispielsweise nach einem bestimmten Zeitraum nach dem letzten Aktivierungssignal. Damit steht dem Operateur nach Erzeugung eines Aktivierungssignals nur ein bestimmtes Zeitfenster zur Verfügung, innerhalb dessen die Bewegung des Fußes zu Schaltsignalen führen kann.

Die beschriebene Signalerzeugung kann an jeder Stelle der Fußmatte erfolgen, der Operateur kann also an jeder Stelle der Fußmatte eine Aktivierung vornehmen, an dieser Stelle Schaltsignale erzeugen und dann nach Deaktivierung sich frei auf der Fußmatte bewegen, ohne dass unerwünschte Schaltsignale entstehen. Ein großer Vorteil dieser Anordnung liegt auch darin, dass die Fußmatte mit glatten Außenflächen hergestellt werden kann und daher reinigungsfreundlich ist.

## Patentansprüche

1. Schalteinrichtung für medizinische oder chirurgische Geräte, wobei die Schalteinrichtung eine flächige Fußmatte (2) mit über ihre Fläche verteilten Sensoren (3) aufweist, die ein der Position eines Fußes (4) einer Bedienungsperson auf der Fußmatte (2) entsprechendes elektrisches Signal erzeugen, wenn sich der Fuß (4) bei dem jeweiligen Sensor (3) befindet, und wobei die Schalteinrichtung eine mit der Fußmatte (2) verbundene Datenverarbeitungseinrichtung (6) ebenfalls aufweist, **dadurch gekennzeichnet, dass** die Datenverarbeitungseinrichtung (6) so programmiert ist, dass sie bei Aktivierung durch ein Aktivierungssignal die momentane Position des Fußes (4) auf der Fußmatte (2) als Ausgangsposition speichert, mindestens einen Schaltbereich (8, 9, 10, 11, 12) der Fußmatte (2), der relativ zu der Ausgangsposition eine vorgegebene Lage aufweist, auswählt und ein Schaltsignal erzeugt, wenn sich der Fuß (4) bei einem Sensor (3) in dem Schaltbereich (8, 9, 10, 11, 12) befindet.

2. Schalteinrichtung nach Anspruch 1, **dadurch gekennzeichnet, dass** sie eine Anzeigeeinrichtung (7) aufweist, die den Schaltbereich oder gegebenenfalls die Schaltbereiche (8, 9, 10, 11, 12) und die jeweilige momentane Position (14) des Fußes (4) der Bedienungsperson anzeigt.

3. Schalteinrichtung nach Anspruch 2, **dadurch gekennzeichnet, dass** der Schaltbereich oder gegebenenfalls die Schaltbereiche (8, 9, 10, 11, 12) in einem Ausschnitt (13) der Anzeige (7) dargestellt werden und dass die Lage des Ausschnittes (13) auf der Anzeige (7) der durch die Ausgangsposition definierten Lage der Schaltbereiche (8, 9, 10, 11, 12) auf der Fußmatte (2) entspricht.

4. Schalteinrichtung nach einem der voranstehenden Ansprüche, **dadurch gekennzeichnet, dass** ein Verschiebeschaltbereich (11) vorgesehen ist, der ein Verschiebesignal erzeugt, wenn sich der Fuß (4) der Bedienungsperson von diesem Verschiebeschaltbereich (11) ausgehend auf der Fußmatte (2) verschiebt, und dass die Datenverarbeitungseinrichtung (6) die Ausgangsposition und damit die Schaltbereiche (8, 9, 10, 11, 12) entsprechend der Bewegung des Fußes (4) von dem Verschiebeschaltbereich (11) an eine andere Stelle der Fußmatte (2) verschiebt und mit den neuen Positionsdaten speichert.

5. Schalteinrichtung nach einem der voranstehenden Ansprüche, **dadurch gekennzeichnet, dass** ein Schaltbereich (12) vorgesehen ist, der ein Deaktivierungssignal erzeugt, durch das die Erzeugung von Schaltsignalen beendet wird.

6. Schalteinrichtung nach einem der voranstehenden Ansprüche, **dadurch gekennzeichnet, dass** die Datenverarbeitungseinrichtung (6) nach Ablauf einer vorgegebenen Zeitspanne ausgehend von dem Aktivierungssignal ein Deaktivierungssignal erzeugt, durch das die Erzeugung von Schaltsignalen beendet wird.

7. Schalteinrichtung nach einem der voranstehenden Ansprüche, **dadurch gekennzeichnet, dass** das Aktivierungssignal von einem akustischen Sensor erzeugt wird.

8. Schalteinrichtung nach einem der Ansprüche 1 bis 6, **dadurch gekennzeichnet, dass** das Aktivierungssignal von der Datenverarbeitungseinrichtung (6) erzeugt wird, wenn ihr von einem einzigen Sensor (3) an einer beliebigen Stelle der Fußmatte (2) nacheinander Signale in einer bestimmten Folge zugeführt werden.

9. Schalteinrichtung nach Anspruch 8, **dadurch gekennzeichnet, dass** diese Folge aus zwei Signalen innerhalb eines kurzen Zeitraumes besteht.

10. Schalteinrichtung nach einem der voranstehenden Ansprüche, **dadurch gekennzeichnet, dass** die Datenverarbeitungseinrichtung (6) nach dem Aktivierungssignal nur Signale von Sensoren (3) berücksichtigt, bei denen sich nach dem Aktivierungssignal die Anwesenheit des Fußes (4) der Bedienungsperson geändert hat.

11. Schalteinrichtung nach einem der voranstehenden Ansprüche, **dadurch gekennzeichnet, dass** die Sensoren (3) in der Fußmatte (2) Drucksensoren sind.

12. Schalteinrichtung nach einem der Ansprüche 1 bis 10, **dadurch gekennzeichnet, dass** die Sensoren (3) in der Fußmatte (2) Ultraschallsensoren sind.

13. Schalteinrichtung nach einem der Ansprüche 1 bis 10, **dadurch gekennzeichnet, dass** die Sensoren (3) in der Fußmatte (2) kapazitive Sensoren sind.

14. Schalteinrichtung nach einem der Ansprüche 1 bis 10, **dadurch gekennzeichnet, dass** die Sensoren (3) in der Fußmatte (2) Hall-Sensoren sind.

15. Schalteinrichtung nach einem der Ansprüche 1 bis 10, **dadurch gekennzeichnet, dass** die Sensoren (3) in der Fußmatte (2) analog-resistive Sensoren sind.

16. Schalteinrichtung nach einem der voranstehenden Ansprüche, **dadurch gekennzeichnet, dass** die Sensoren (3) auf die Anwesenheit von Schaltelementen (15) ansprechen und dass ein solches Schaltelement (15) an einem Fuß (4) der Bedienungsperson angeordnet ist.

17. Schalteinrichtung nach einem der voranstehenden Ansprüche, **dadurch gekennzeichnet, dass** die Größe der Schaltbereiche (8, 9, 10, 11, 12) auf der Fußmatte (2) und/oder deren relative Lage zur Ausgangsposition einstellbar sind.

18. Schalteinrichtung nach Anspruch 17, **dadurch gekennzeichnet, dass** ein Einstellschaltbereich (11) vorgesehen ist, der ein Verschiebesignal erzeugt, wenn sich der Fuß (4) der Bedienungsperson von diesem Einstellchaltbereich (11) ausgehend auf der Fußmatte (2) verschiebt, und dass die Datenverarbeitungseinrichtung (6) die Größe der Schaltbereiche (8, 9, 10, 11, 12) und/oder deren relative Lage zur Ausgangsposition entsprechend der Bewegung des Fußes (4) von dem Einstellschaltbereich (11) an eine andere Stelle der Fußmatte (2) verstellt.

19. Schalteinrichtung nach einem der voranstehenden Ansprüche, **dadurch gekennzeichnet, dass** ein Einstellschaltbereich (11) vorgesehen ist, der ein Verschiebesignal erzeugt, wenn sich der Fuß (4) der Bedienungsperson von diesem Einstellschaltbereich (11) ausgehend auf der Fußmatte (2) verschiebt, und dass die Datenverarbeitungseinrichtung (6) die Größe eines Parameters eines chirurgischen Gerätes entsprechend der Bewegung des Fußes (4) von dem Einstellschaltbereich (11) an eine andere Stelle der Fußmatte (2) verstellt.

20. Schalteinrichtung nach einem der voranstehenden Ansprüche, **dadurch gekennzeichnet, dass** die Datenverarbeitungseinrichtung (6) derart programmiert ist, dass sie den oder die Schaltbereiche (8, 9, 10, 11, 12) entsprechend der Verschiebung des Fußes (4) in eine neue momentane Position auf der Fußmatte (2) verschiebt, wenn der Fuß (4) über einen bestimmten Zeitraum an der neuen momentanen Position verweilt.

21. Schalteinrichtung nach einem der voranstehenden Ansprüche, **dadurch gekennzeichnet, dass** die Datenverarbeitungseinrichtung (6) eine Auswahleinrichtung umfasst zur selektiven Ansteuerung und Versorgung mit Schalt- und/oder Einstellsignalen verschiedener chirurgischer Geräte.

22. Schalteinrichtung nach Anspruch 21, **dadurch gekennzeichnet, dass** die Datenverarbeitungseinrichtung (6) für verschiedene chirurgische Geräte verschiedene Anordnungen, Größen und/ oder Anzahl von Schaltbereichen (8, 9, 10, 11, 12) auf der Fußmatte (2) definiert.

23. Schalteinrichtung nach einem der Ansprüche 21 oder 22, **dadurch gekennzeichnet, dass** der Auswahleinrichtung ein oder mehrere eigene Schaltbereiche auf der Fußmatte zugeordnet sind.

## Claims

1. A switching device for medical or surgical equipment, the switching device comprising **characterized by** a planar floor mat (2) having sensors (3) distributed over the surface area thereof, said sensors producing an electrical signal corresponding to the position of a foot (4) of an operator on the floor mat (2) when the foot (4) is located near the respective sensor (3), and the switching device also comprising a data processing system (6) which is connected to the floor mat (2), **characterised in that** the data processing system (6) is programmed in such a manner that it stores the instantaneous position of the foot (4) on the floor mat (2) as a starting position when activated by an activating signal, selects at least one switching area (8, 9, 10, 11, 12) of the floor mat (2) which has a predetermined location relative to the starting position, and produces a switching signal when the foot (4) is located near a sensor (3) in the switching area (8, 9, 10, 11, 12).

2. A switching device in accordance with Claim 1, **characterised in that** it comprises a display device (7) which indicates the switching area or, if applicable, the switching areas (8, 9, 10, 11, 12) and the respective instantaneous position (14) of the foot (4) of the operator.

3. A switching device in accordance with Claim 2, **characterised in that** the switching area or, if applicable, the switching areas (8, 9, 10, 11, 12) are represented in a section (13) of the display means (7) and **in that** the location of the section (13) on the display means (7) corresponds to the location of the switching areas (8, 9, 10, 11, 12) on the floor mat (2) that is defined by the starting position.

4. A switching device in accordance with any of the preceding Claims, **characterised in that** there is provided a displacement switching area (11) which produces a displacement signal if the foot (4) of the operator is displaced on the floor mat (2) commencing from this displacement switching area (11), and **in that** the data processing system (6) shifts the starting position and thus the switching areas (8, 9, 10, 11, 12) in correspondence with the movement of the foot (4) from the displacement switching area (11) to another location on the floor mat (2) and stores these together with the new positional data.

5. A switching device in accordance with any of the preceding Claims, **characterised in that** there is provided a switching area (12) which produces a deactivating signal by means of which the production of switching signals is terminated.

6. A switching device in accordance with any of the preceding Claims, **characterised in that**, after the expiry of a given time interval commencing from the activating signal, the data processing system (6) produces a deactivating signal by means of which the production of switching signals is terminated.

7. A switching device in accordance with any of the preceding Claims, **characterised in that** the activating signal is produced by an acoustic sensor.

8. A switching device in accordance with any of the Claims 1 to 6, **characterised in that** the activating signal is produced by the data processing system (6) if a certain sequence of successive signals is supplied thereto from a single sensor (3) at an arbitrary location on the floor mat (2).

9. A switching device in accordance with Claim 8, **characterised in that** this sequence consists of two signals within a short time period.

10. A switching device in accordance with any of the preceding Claims, **characterized in that**, following the activating signal, the data processing system (6) only takes into account signals from sensors (3) at which the presence of the foot (4) of the operator has changed after the activating signal.

11. A switching device in accordance with any of the preceding Claims, **characterised in that** the sensors (3) are pressure sensors in the floor mat (2).

12. A switching device in accordance with any of the Claims 1 to 10, **characterised in that** the sensors (3) are ultrasonic sensors in the floor mat (2).

13. A switching device in accordance with any of the Claims 1 to 10, **characterised in that** the sensors (3) are capacitive sensors in the floor mat (2).

14. A switching device in accordance with any of the Claims 1 to 10, **characterised in that** the sensors (3) are Hall sensors in the floor mat (2).

15. A switching device in accordance with any of the Claims 1 to 10, **characterised in that** the sensors (3) are analogue resistive sensors in the floor mat (2).

16. A switching device in accordance with any of the preceding Claims, **characterised in that** the sensors (3) respond to the presence of switching elements (15) and **in that** such a switching element (15) is arranged on a foot (4) of the operator.

17. A switching device in accordance with any of the preceding Claims, **characterised in that** the size of the switching areas (8, 9, 10, 11, 12) on the floor mat (2) and/or their relative location with respect to the starting position are adjustable.

18. A switching device in accordance with Claim 17, **characterised in that** there is provided an adjustment switching area (11) which produces a displacement signal if the foot (4) of the operator is displaced from this adjustment switching area (11) of the floor mat (2), and **in that** the data processing system (6) adjusts the size of the switching areas (8, 9, 10, 11, 12) and/or their relative location with respect to the starting position in correspondence with the movement of the foot (4) from the adjustment switching area (11) to another location on the floor mat (2).

19. A switching device in accordance with any of the preceding Claims, **characterised in that** there is provided an adjustment switching area (11) which produces a displacement signal if the foot (4) of the operator is displaced from this adjustment switching area (11) of the floor mat (2), and **in that** the data processing system (6) adjusts the magnitude of a parameter of a surgical equipment in correspondence with the movement of the foot (4) from the adjustment switching area (11) to another location on the floor mat (2).

20. A switching device in accordance with any of the preceding Claims, **characterised in that** the data processing system (6) is programmed in such a manner that it shifts the switching area or the switching areas (8, 9, 10, 11, 12) into a new instantaneous position on the floor mat (2) in correspondence with the displacement of the foot (4) if the foot (4) stays at the new instantaneous position for a certain period of time.

21. A switching device in accordance with any of the preceding Claims, **characterised in that** the data processing system (6) comprises a selecting device for the selective control of various surgical equipments and the supply of switching and/or adjusting signals thereto.

22. A switching device in accordance with Claim 21, **characterised in that** the data processing system (6) defines different arrangements, sizes and/or numbers of switching areas (8, 9, 10, 11, 12) on the floor mat (2) for various surgical equipments.

23. A switching device in accordance with either of the Claims 21 or 22, **characterised in that** one or more individual switching areas of the floor mat are assigned to the selecting device.

## Revendications

1. Système de commande pour appareils médicaux ou chirurgicaux, le système de commande présentant un tapis de sol (2) plan comprenant, répartis sur sa surface, des détecteurs (3) qui produisent un signal électrique correspondant à la position d'un pied (4) d'une personne de service sur le tapis de sol (2), lorsque le pied (4) se trouve au niveau du détecteur (3) respectif, et le système de commande présentant également un appareillage de traitement de données (6) relié au tapis de sol (2), **caractérisé en ce que** l'appareillage de traitement de données (6) est programmé de manière à mémoriser, en cas d'activation par un signal d'activation, la position momentanée du pied (4) sur le tapis de sol (2) en tant que position initiale, à sélectionner au moins une zone de commande (8, 9, 10, 11, 12) du tapis de sol (2), qui présente une position prédéterminée par rapport à la position initiale, et à produire un signal de commande lorsque le pied (4) se trouve au niveau d'un détecteur (3) dans la zone de commande (8, 9, 10, 11, 12).

2. Système de commande selon la revendication 1, **caractérisé en ce qu'**il comporte un système d'affichage (7) qui affiche la zone de commande ou, le cas échéant, les zones de commande (8, 9, 10, 11, 12) et la position momentanée (14) respective du pied (4) de la personne de service.

3. Système de commande selon la revendication 2, **caractérisé en ce que** la zone de commande ou, le cas échéant, les zones de commande (8, 9, 10, 11, 12) sont représentées sur un secteur (13) de l'affichage (7), et **en ce que** la position du secteur (13) sur l'affichage (7) correspond à la position des zones de commande (8, 9, 10, 11, 12) sur le tapis de sol (2), définie par la position initiale.

4. Système de commande selon l'une des revendications précédentes, **caractérisé en ce qu'**il est prévu une zone de commande de translation (11), qui produit un signal de translation lorsque le pied (4) de la personne de service est translaté, à partir de cette zone de commande de translation (11), sur le tapis de sol (2), et **en ce que** l'appareillage de traitement de données (6) translate la position initiale et ainsi les zones de commande (8, 9, 10, 11, 12), conformément au mouvement du pied (4), de la zone de commande de translation (11) à un autre endroit du tapis de sol (2), et la mémorise avec les nouvelles données de position.

5. Système de commande selon l'une des revendications précédentes, **caractérisé en ce qu'**il est prévu une zone de commande (12), qui produit un signal de désactivation arrêtant la production de signaux de commande.

6. Système de commande selon l'une des revendications précédentes, **caractérisé en ce que** l'appareillage de traitement de données (6), après écoulement d'un intervalle de temps prédéterminé à partir du signal d'activation, produit un signal de désactivation arrêtant la production de signaux de commande.

7. Système de commande selon l'une des revendications précédentes, **caractérisé en ce que** le signal d'activation est produit par un détecteur acoustique.

8. Système de commande selon l'une des revendications 1 à 6, **caractérisé en ce que** le signal d'activation est produit par l'appareillage de traitement de données (6)
lorsque lui sont transmis successivement, par un seul détecteur (3) en un endroit quelconque du tapis de sol (2), des signaux selon une suite déterminée.

9. Système de commande selon la revendication 8, **caractérisé en ce que** cette suite est constituée de deux signaux en l'espace d'un court intervalle de temps.

10. Système de commande selon l'une des revendications précédentes, **caractérisé en ce qu'**après le signal d'activation, l'appareillage de traitement de données (6) ne prend en compte que les signaux de détecteurs (3) pour lesquels la présence du pied (4) de la personne de service s'est modifiée après le signal d'activation.

11. Système de commande selon l'une des revendications précédentes, **caractérisé en ce que** les détecteurs (3) dans le tapis de sol (2) sont des détecteurs de pression.

12. Système de commande selon l'une des revendications 1 à 10, **caractérisé en ce que** les détecteurs (3) dans le tapis de sol (2) sont des détecteurs à ultrasons.

13. Système de commande selon l'une des revendications 1 à 10, **caractérisé en ce que** les détecteurs (3) dans le tapis de sol (2) sont des détecteurs capacitifs.

14. Système de commande selon l'une des revendications 1 à 10, **caractérisé en ce que** les détecteurs (3) dans le tapis de sol (2) sont des détecteurs de Hall.

15. Système de commande selon l'une des revendications 1 à 10, **caractérisé en ce que** les détecteurs (3) dans le tapis de sol (2) sont des détecteurs analogiques-résistifs.

16. Système de commande selon l'une des revendications précédentes, **caractérisé en ce que** les détecteurs (3) réagissent à la présence d'éléments de commande (15), et **en ce qu'**un tel élément de commande (15) est agencé sur un pied (4) de la personne de service.

17. Système de commande selon l'une des revendications précédentes, **caractérisé en ce que** la grandeur des zones de commande (8, 9, 10, 11, 12) sur le tapis de sol (2) et/ou leur position relative par rapport à la position initiale sont réglables.

18. Système de commande selon la revendication 17, **caractérisé en ce qu'**il est prévu une zone de commande de réglage (11), qui produit un signal de translation lorsque le pied (4) de la personne de service se déplace à partir de cette zone de commande de réglage (11) sur le tapis de sol (2), et **en ce que** l'appareillage de traitement de données (6) règle la grandeur des zones de commande (8, 9, 10, 11, 12) et/ou leur position relative par rapport à la position initiale, conformément au mouvement du pied (4) de la zone de commande de réglage (11) à un autre endroit du tapis de sol (2).

19. Système de commande selon l'une des revendications précédentes, **caractérisé en ce qu'**il est prévu une zone de commande de réglage (11), qui produit un signal de translation lorsque le pied (4) de la personne de service se déplace à partir de cette zone de commande de réglage (11) sur le tapis de sol (2), et **en ce que** l'appareillage de traitement de données (6) règle la grandeur d'un paramètre d'un appareil chirurgical, conformément au mouvement du pied (4) de la zone de commande de réglage (11) à un autre endroit du tapis de sol (2).

20. Système de commande selon l'une des revendications précédentes, **caractérisé en ce que** l'appareillage de traitement de données (6) est programmé de manière à translater la ou les zones de commande (8, 9, 10, 11, 12) dans une nouvelle position momentanée sur le tapis de sol (2), conformément à la translation du pied (4), lorsque le pied (4) séjourne pendant une durée déterminée dans la nouvelle position momentanée.

21. Système de commande selon l'une des revendications précédentes, **caractérisé en ce que** l'appareillage de traitement de données (6) comprend un dispositif de sélection pour la commande sélective de différents appareils chirurgicaux et leur alimentation en signaux de commande et/ou de réglage.

22. Système de commande selon la revendication 21, **caractérisé en ce que** l'appareillage de traitement de données (6) définit pour différents appareils chirurgicaux, différents agencements, grandeurs et/ou nombres de zones de commande (8, 9, 10, 11, 12) sur le tapis de sol (2).

23. Système de commande selon l'une des revendications 21 ou 22, **caractérisé en ce qu'**au dispositif de sélection sont associées une ou plusieurs zones de commande, qui lui sont propres, sur le tapis de sol.
